(19) Europäisches Patentamt
European Patent Office
Office européen des brevets

(11) Veröffentlichungsnummer: **0 000 018**
**B1**

(12) # EUROPÄISCHE PATENTSCHRIFT

(45) Veröffentlichungstag der Patentschrift: **07.01.81**

(21) Anmeldenummer: **78100030.2**

(22) Anmeldetag: **01.06.78**

(51) Int. Cl.³: **C 07 D 249/08**, A 01 N 43/64, C 07 F 1/00, C 07 F 3/00, C 07 F 15/00

(54) **Metallsalzkomplexe von 1-Phenyl-2-triazolyl-ethyl-Derivaten, Verfahren zu ihrer Herstellung sowie ihre Verwendung als Fungizide.**

(30) Priorität: **03.06.77 DE 2725214**

(43) Veröffentlichungstag der Anmeldung: **20.12.78 Patentblatt 78/1**

(45) Bekanntmachung des Hinweises auf die Patenterteilung: **07.01.81 Patentblatt 81/1**

(84) Benannte Vertragsstaaten: **BE CH DE FR GB NL SE**

(56) Entgegenhaltungen: **Keine**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal 1 (DE)**
Erfinder: **Timmler, Helmut, Dr., Tersteegenweg 16, D-5600 Wuppertal 11 (DE)**
Erfinder: **Büchel, Karl Heinz, Dr., Bergerheide 62, D-5600 Wuppertal 1 (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen (DE)**
Erfinder: **Scheinpflug, Hans, Dr., Am Thelenhof 15, D-5090 Leverkusen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchgebühr entrichtet worden ist (Art. 99(1) Europäisches Patentübereinkommen).



ACTORUM AG.

# Metallsalzkomplexe von 1-Phenyl-2-triazolyl-ethyl-Derivaten, Verfahren zu ihrer Herstellung sowie ihre Verwendung als Fungizide

Die Erfindung betrifft neue Metallsalzkomplexe von 1-Phenyl-2-triazolyl-ethyl-derivaten, ein Verfahren zu ihrer Herstellung sowie ihre Verwendung als Fungizide.

Es ist bereits bekannt geworden, dass 1-[β-Aryl-β-(R-oxy)-ethyl]-imidazole, wie z.B. 1-[β-Butoxy-β-(4'-chlorphenyl)-ethyl]-imidazol und 1-[β-Aryl-β-(R-oxy)-ethyl]-triazole, wie z.B. 1-[β-Allyloxy-β-(4'-chlorphenyl)-ethyl]-1,2,4-triazol, eine gute fungizide Wirksamkeit aufweisen (vgl. Deutsche Offenlegungsschriften 2063857 und 2640823). Deren Wirkung ist jedoch in bestimmten Indikationsbereichen, insbesondere bei niedrigen Aufwandmengen und -konzentrationen, nicht immer voll befriedigend. Weiterhin ist allgemein seit längerer Zeit bekannt, dass Zink-ethylen-1,2-bisdithiocarbamidat ein gutes Mittel zur Bekämpfung von pilzlichen Pflanzenkrankheiten ist [vgl. Phytopathology 33, 1113 (1963)]. Jedoch ist dessen Einsatz als Saatgutbeizmittel nur beschränkt möglich, da es bei niedrigen Aufwandmengen und -konzentrationen wenig wirksam ist.

Es wurden nun als neue Verbindungen die Metallsalzkomplexe von 1-Phenyl-2-triazolyl-ethyl-Derivaten der Formel I

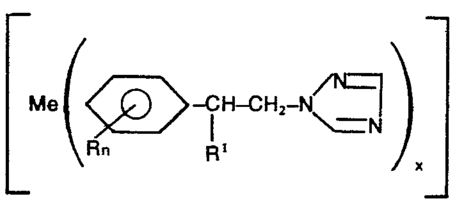 (I)

in welcher

R für Fluor, Chlor, Brom und für Alkyl mit 1 bis 4 Kohlenstoffatomen steht, ferner für gegebenenfalls durch Fluor, Chlor oder Brom substituiertes Phenyl oder Phenoxy steht,

$R^1$ für die Gruppierungen $-O-R^2$ oder $-O-CO-R^3$ steht, wobei

$R^2$ und $R^3$ für Alkyl, Alkenyl und Alkinyl mit jeweils bis zu 4 Kohlenstoffatomen, ferner für gegebenenfalls im Arylteil durch Fluor, Chlor, Brom oder Alkyl mit 1 bis 5 Kohlenstoffatomen substituiertes Benzyl stehen,

A für ein Chlorid-, Nitrat- oder Sulfat-Anion steht,

Me für die Metalle Kupfer, Zink, Mangan, Magnesium, Zinn, Eisen und Nickel steht,

n für ganze Zahlen von 0 bis 3 steht,

p für ganze Zahlen von 1 bis 4 steht und

x für ganze Zahlen von 1 bis 4 steht,

gefunden. Sie weisen starke fungizide Eigenschaften auf.

Weiterhin wurde gefunden, dass man die Metallkomplexe der Formel I erhält, wenn man 1-Phenyl-2-triazolyl-ethyl-Derivate der Formel II

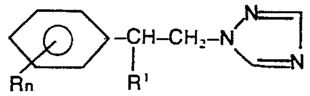 (II)

in welcher

R, $R^1$ und n die oben angegebene Bedeutung haben,

mit Metallsalzen der Formel III

$$MeA_p \times k\,H_2O \qquad (III)$$

in welcher

k für beliebige Zahlen von 0 bis 12 steht und Me, A und p die oben angegebene Bedeutung haben,

in Gegenwart eines Lösungsmittels umsetzt.

Überraschenderweise zeigen die erfindungsgemässen Metallkomplexe von 1-Phenyl-2-triazolyl-ethyl-Derivaten eine erheblich höhere fungizide Wirksamkeit, insbesondere gegen Rost- und Mehltauarten, als die aus dem Stand der Technik bekannten 1-[β-Aryl-β(R-oxy)-ethyl]-imidazole und -triazole, beispielsweise 1-[β-Butoxy-β-(4'-chlorphenyl)-ethyl]-imidazol, welche chemisch und wirkungsmässig naheliegende Stoffe darstellen, und als das Zink-ethylen-1,2-bis-dithiocarbamidat, welches ein bekannter Stoff gleicher Wirkungsrichtung ist. Die erfindungsgemässen Wirkstoffe stellen somit eine Bereicherung der Technik dar.

Verwendet man [1-(2,4-Dichlorphenyl)-2-(1,2,4-triazol-1-yl)-ethyl]-allyl-ether und Kupfer(II)-chlorid als Ausgangsstoffe, so kann der Reaktionsablauf durch das folgende Formelschema wiedergegeben werden:

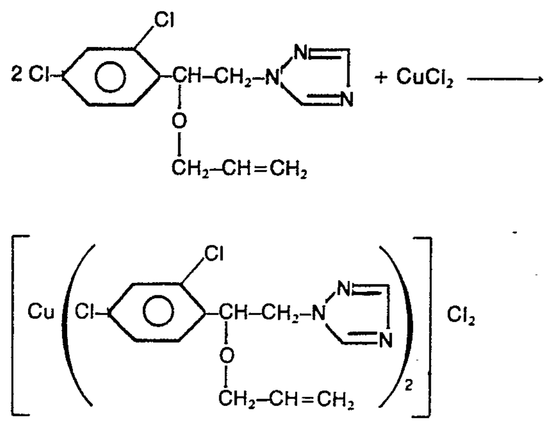

Die als Ausgangsstoffe zu verwendenden 1-Phenyl-2-triazolyl-ethyl-Derivate sind durch die Formel II allgemein definiert.

Die Ausgangsstoffe der Formel II sind teilweise bekannt (vgl. DT-OS 2547953 und DT-OS 2640823), teilweise sind sie Gegenstand eigener, älterer Anmeldungen, die noch nicht zum Stand der Technik gehören (vgl. die Deutschen Patentanmeldungen P 2628419 vom 24.6.1976 und P 2645496 vom 8.10.1976). Noch nicht bekannte Ausgangsstoffe der Formel II können nach den dort beschriebenen Verfahren erhalten werden, indem man z.B.

a) Alkanolate von 1-Hydroxy-1-phenyl-2-triazolyl-ethan-Derivaten der Formel IV

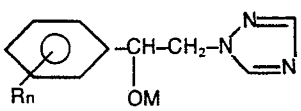 (IV)

in welcher

R und n die oben angegebene Bedeutung haben und

M für ein Alkalimetall, vorzugsweise Lithium, Natrium und Kalium, eine quarternäre Ammonium- oder Phosphoniumgruppe steht,

mit einem Halogenid der Formel V

Hal-$R^1$ (V)

in welcher

$R^1$ die oben angegebene Bedeutung hat und

Hal für Chlor oder Brom steht,

in Gegenwart eines organischen Lösungsmittels, wie beispielsweise Dioxan oder Chloroform, bei Temperaturen zwischen 20 und 120°C umsetzt. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmässigerweise so verfahren, dass man von einem 1-Hydroxy-1-phenyl-2-triazolyl-ethan-Derivat ausgeht, letzteres in einem geeigneten inerten Lösungsmittel mittels Alkalimetall-hydrid oder Alkalimetallamid in das Alkalimetall-alkoholat der Formel IV überführt, und letzteres ohne Isolierung sofort mit einem Halogenid der Formel V umsetzt, wobei unter Austritt von Alkalihalogenid die Verbindungen der Formel II in einem Arbeitsgang erhalten werden.

Nach einer weiteren bevorzugten Ausführungsform werden zweckmässigerweise die Herstellung der Alkanolate der Formel IV sowie die Umsetzung mit dem Halogenid der Formel V in einem Zweiphasensystem, wie beispielsweise wässrige Natron- oder Kalilauge/Toluol oder Methylenchlorid, unter Zusatz eines Phasen-Transfer-Katalysators, wie Beispielsweise Ammonium- oder Phosphoniumverbindungen, durchgeführt.

Die Ausgangsstoffe der Formel II können auch erhalten werden, wenn man

b) die den Alkanolaten der Formel IV zugrunde liegenden 1-Hydroxy-1-phenyl-2-triazolyl-ethane

- mit entsprechenden Säureanhydriden nach bekannten Methoden, z.B. in molaren Mengen in Gegenwart eines inerten organischen Lösungsmittels, beispielsweise Aceton, oder mit einem Überschuss an Säureanhydrid und in Gegenwart eines sauren oder basischen Katalysators, beispielsweise Natriumacetat, bei Temperaturen zwischen 0 und 150°C umsetzt und die Verbindungen der Formel II in üblicher Weise isoliert, oder
- mit entsprechenden Isocyanaten nach bekannten Methoden, z.B. in molaren Mengen in Gegenwart eines inerten organischen Lösungsmittels, beispielsweise Benzol, und in Gegenwart eines Katalysators, beispielsweise Dibutylzinndilaurat, bei Temperaturen zwischen 0 und 100°C umsetzt und die Verbindungen der Formel II in üblicher Weise isoliert.

Die den Alkanolaten der Formel IV zugrunde liegenden 1-Hydroxy-1-phenyl-2-triazolyl-ethan-Derivate sind bekannt (vgl. DT-OS 2431407 und DT-OS 2547953).

Die 1-Halogen-1-phenyl-2-triazolyl-ethan-Derivate der Formel VI sind ebenfalls bekannt (vgl. DT-OS 2547954).

Als Beispiele für die erfindungsgemäss als Ausgangsstoffe zu verwendenden 1-Phenyl-2-triazolyl-ethyl-Derivate der Formel II seien im einzelnen folgende Verbindungen der Tabellen 1, 2 und 3 genannt:

Tabelle 1

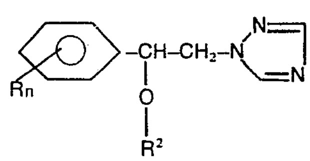 (IIa)

| $R_n$ | $R^2$ | Schmelzpunkt (°C) bzw. Brechungsindex |
|---|---|---|
| 2,4-$Cl_2$ | 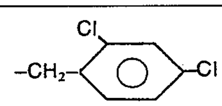 | 84 |
| 2,4-$Cl_2$ | $-CH_2-CH=CH_2$ | $n_D^{20}$ = 1,545 |
| 2,4-$Cl_2$ | $-CH_2-C\equiv$ | 130(Zers.)(x$HNO_3$) |
| 4-Cl | 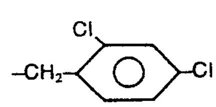 | 78 |

Tabelle 1 (Fortsetzung)

| $R_n$ | $R^2$ | Schmelzpunkt (°C) bzw. Brechungsindex |
|---|---|---|
| 4-Cl | 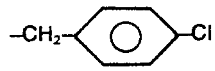 | 111 |
| 2,4-$Cl_2$ | 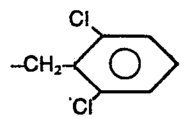 | 120 |
| 4-Cl | 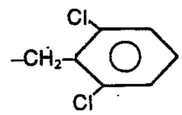 | 118 |
| 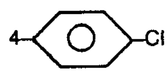 | $-CH_2-CH=CH_2$ | 225(Zers.)(x½ NDS) |
| 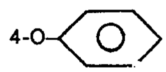 | $-CH_2-CH=CH_2$ | $n^{2}_{D}{}^{2}$= 1,570 |
| 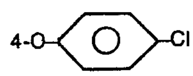 | 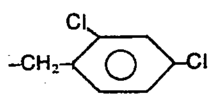 | 200 (x½ NDS) |
| 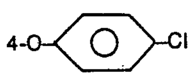 | $-CH_2-CH=CH_2$ | 213 (x½ NDS) |
| 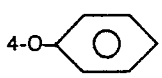 | 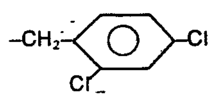 | 145 (Zers.)(x½ NDS) |
| 2,4-$Cl_2$ | $-CH_3$ | 143-46 (x HCl) |
| 4-Br | $-CH_2-CH=CH_2$ | 193 (x½ NDS) |
| 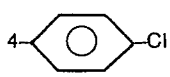 | 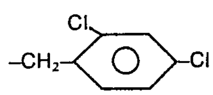 | 237 (x½ NDS) |
| 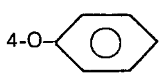 | 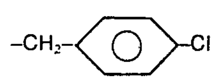 | 90 |
| 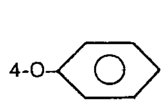 | 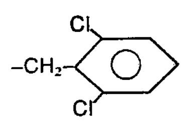 | 124 |
| 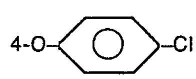 | $-CH_3$ | 211(Zers.)(x½ NDS) |
| 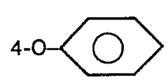 | $-CH_2-C\equiv CH$ | 158(Zers.)(x½ NDS) |
| 4-Br | 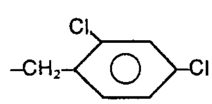 | 76 |
| 4-Cl | $-CH_2-CH=CH_2$ | 140 (x HCl) |

Tabelle 1 (Fortsetzung)

| $R_n$ | $R^2$ | Schmelzpunkt (°C) bzw. Brechungsindex |
|---|---|---|
| 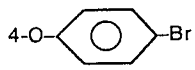 | 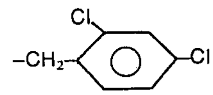 | zähfl. Öl |
| 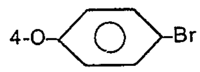 | $-CH_2-CH=CH_2$ | zähfl. Öl |
| 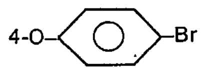 | 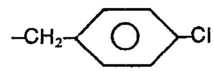 | 135 |
| 2,4-$Cl_2$ | 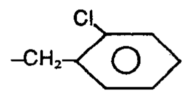 | 108 |
| 2,4-$Cl_2$ | $-C_4H_9$ | zähfl. Öl |
| 2,4-$Cl_2$ | 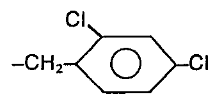 | 89-91 |
| 2,4-$Cl_2$ | 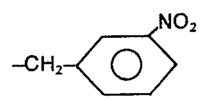 | 105 |
| 4-Br | 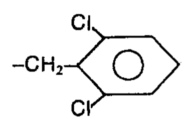 | 138 |
| 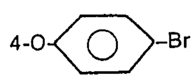 | 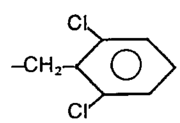 | 189 (x HCl) |
| 4-Br | 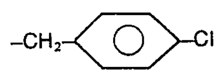 | 102 |
| 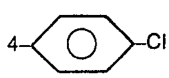 | 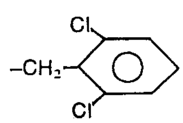 | 165 |
| 2,4-$Cl_2$ | 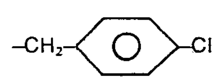 | 107 |
| 2,4-$Cl_2$ | $-CH_2-C=CH_2$<br>\|<br>$CH_3$ | zähfl. Öl |
| 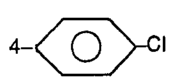 | 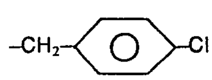 | 103 |
| 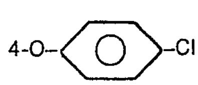 | 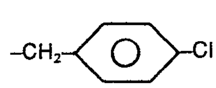 | 136 |
| 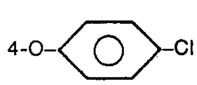 | 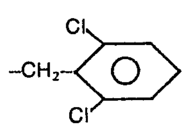 | 98 |

Tabelle 1 (Fortsetzung)

| $R_n$ | $R^2$ | Schmelzpunkt (°C) bzw. Brechungsindex |
|---|---|---|
| 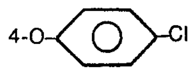 | $-CH_2-C=CH_2$<br>\|<br>$CH_3$ | 185(Zers.)(x½ NDS) |
| 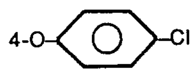 | $-CH_2-C\equiv CH$ | zähfl. Öl |
| 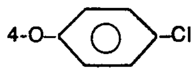 | 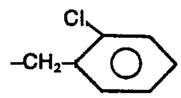 | 212 (x½ NDS) |
| 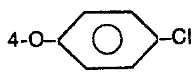 | 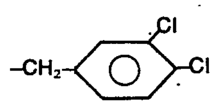 | 212 (x½ NDS) |
| 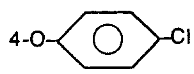 | 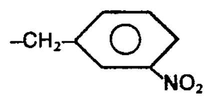 | 203 |
| 2,4-$Cl_2$ | 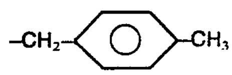 | 92 |
| 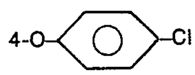 | 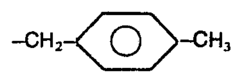 | 214 (x½ NDS) |

NDS = 1,5-Naphthalindisulfonsäure

Tabelle 2

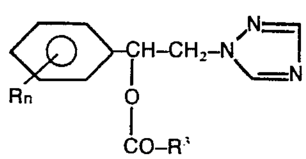

(IIb)

| $R_n$ | $R^3$ | Schmelzpunkt (°C) |
|---|---|---|
| 2,4-$Cl_2$ | $-C(CH_3)_3$ | 149–51 (× $HNO_3$) |
| 2,4-$Cl_2$ | $-CH_3$ | 92–96 |
| 2,4-$Cl_2$ | $-C_2H_5$ | 156 (x½ NDS) |
| 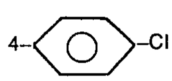 | $-CH_3$ | 123 |
| 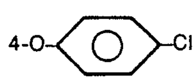 | $-CH_3$ | 150 (× $HNO_3$) |
| 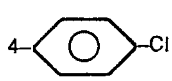 | $-C(CH_3)_3$ | 121 |
| 4-Cl | $-CH_3$ | 90–92 |
| 4-Cl | $-C(CH_3)_3$ | 135–136 |

NDS = 1,5-Naphthalindisulfonsäure

Die benötigten Metallsalze der Formel III sind allgemein bekannte, leicht zugängliche Verbindungen.

Für die erfindungsgemässe Umsetzung kommen als Verdünnungsmittel Wasser und alle inerten organischen Lösungsmittel in Frage. Hierzu gehören vorzugsweise Alkohole, wie Methanol und Ethanol, Ketone, wie Aceton oder Ethyl-methyl-keton, sowie Ether, wie z.B. Diethyl-ether und Dioxan.

Die Reaktionstemperaturen können in einem grösseren Bereich variiert werden. Im allgemeinen arbeitet man zwischen 0 und 40°C, vorzugsweise zwischen 15 und 30°C.

Bei der Durchführung des erfindungsgemässen Verfahrens setzt man auf 1 Mol des Metallsalzes III die stöchiometrische Menge (je nach der Oxidationsstufe des Metalls) der Verbindung der Formel II ein. Überschreitungen dieser Verhältnisse um bis zu 20 Mol-% können ohne wesentliche Ausbeuteminderung erfolgen. Die Aufarbeitung erfolgt in einer für organische Verbindungen üblichen und allgemein bekannten Weise, z.B. durch Absaugen des ausgefallenen Komplexes und Reinigung durch Umkristallisation, z.B. aus Alkohol.

Die erfindungsgemässen Wirkstoffe weisen eine starke fungitoxische Wirkung auf. Sie schädigen Kulturpflanzen in den zur Bekämpfung von Pilzen notwendigen Konzentrationen nicht. Aus diesen Gründen sind sie für den Gebrauch als Pflanzenschutzmittel zur Bekämpfung von Pilzen geeignet. Fungitoxische Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die erfindungsgemässen Wirkstoffe haben ein breites Wirkungsspektrum und können angewandt werden gegen parasitäre Pilze, die oberirdische Pflanzenteile befallen oder die Pflanzen vom Boden her angreifen, sowie gegen samenübertragbare Krankheitserreger. Eine besonders gute Wirksamkeit entfalten sie gegen parasitäre Pilze auf oberirdischen Pflanzenteilen.

Als Pflanzenschutzmittel können die erfindungsgemässen Wirkstoffe mit besonders gutem Erfolg zur Bekämpfung von Venturia-Arten, wie gegen den Erreger des Apfelschorfs (Fusicladium dendriticum), von echten Mehltaupilzen, beispielsweise zur Bekämpfung von Apfelmehltau (Podosphaera leucotricha) und Getreidemehltau sowie gegen andere Getreidekrankheiten verwendet werden.

Besonders hervorzuheben ist die teilweise systemische Wirkung der Stoffe. So gelingt es, Pflanzen gegen Pilzbefall zu schützen, wenn man den Wirkstoff über den Boden und die Wurzel den oberirdischen Teilen der Pflanze zuführt.

Als Pflanzenschutzmittel können die erfindungsgemässen Wirkstoffe zur Saatgut- oder Bodenbehandlung und zur Behandlung oberirdischer Pflanzenteile benutzt werden.

Die erfindungsgemässen Wirkstoffe können in die üblichen Formulierungen übergeführt werden, wie Lösungen, Emulsionen, Suspensionen, Pulver, Pasten und Granulate. Diese werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/ oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, Benzol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Dichlordifluormethan oder Trichlorfluormethan; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als Emulgiermittel; nichtionogene und anionische Emulgatoren, wie Polyoxyethylen-Fettsäure-Ester, Polyoxyethylen-Fettalkohol-Ether, z.B. Alkylaryl-polyglycol-Ether, Alkylsulfonate, Alkylsulfate, Arylsulfonate sowie Eiweisshydrolysate; als Dispergiermittel: z.B. Lignin, Sulfitablaugen und Methylcellulose.

Die erfindungsgemässen Wirkstoffe können in den Formulierungen in Mischung mit anderen bekannten Wirkstoffen vorliegen, wie Fungiziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Formulierungen enthalten im allgemeinen zwischen 0,1 und 95 Gew.-% Wirkstoff, vorzugsweise zwischen 0,5 und 90%.

Die Wirkstoffe können als solche, in Form ihrer Formulierungen oder der daraus durch weiteres Verdünnen bereiteten Anwendungsformen, wie gebrauchsfertigen Lösungen, Emulsionen, Suspensionen, Pulver, Pasten und Granulate angewendet werden. Die Anwendung geschieht in üblicher Weise, z.B. durch Giessen, Spritzen, Sprühen, Stäuben, Streuen, Trockenbeizen, Feuchtbeizen, Nassbeizen, Schlämmbeizen oder Inkrustieren.

Bei der Verwendung als Blattfungizide können die Wirkstoffkonzentrationen in den Anwendungsformen in einem grösseren Bereich variiert werden. Sie liegen im allgemeinen zwischen 0,1

und 0,00001 Gew.-%. Vorzugsweise zwischen 0,05 und 0,0001%.

Bei der Saatgutbehandlung werden im allgemeinen Wirkstoffmengen von 0,001 bis 50 g je Kilogramm Saatgut, vorzugsweise 0,01 bis 10 g benötigt.

Zur Bodenbehandlung sind Wirkstoffmengen von 1 bis 1000 g je cbm Boden, wie insbesondere 10 bis 200 g erforderlich.

Die vielseitigen Verwendungsmöglichkeiten gehen aus den nachfolgenden Beispielen hervor.

Beispiel A

Fusicladium-Test (Apfel)/Protektiv

Lösungsmittel: 4,7 Gew.-Teile Aceton
Emulgator: 0,3 Gew.-Teile Alkyl-aryl-polyglykol-ether
Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschliessend werden sie mit einer wässrigen Konidiensuspension des Apfelschorferregers (Fusicladium dendriticum) inokuliert und 18 Stunden lang in einer Feuchtkammer bei 18 bis 20°C und 100% relativer Luftfeuchtigkeit inkubiert.

Die Pflanzen kommen dann erneut für 14 Tage ins Gewächshaus.

15 Tage nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Wirkstoffe, Wirkstoffkonzentrationen und Ergebnisse gehen aus der nachfolgenden Tabelle hervor:

Tabelle A

Fusicladium-Test (Apfel)/Protektiv

| Wirkstoff | Befall in % bei einer Wirkstoffkonzentration von 0,0025% |
|---|---|
| 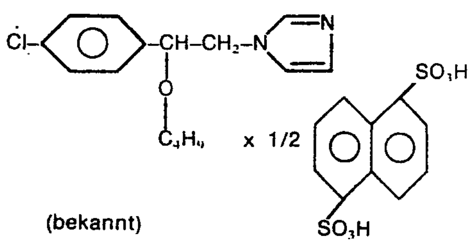 (bekannt) | 51 |
| 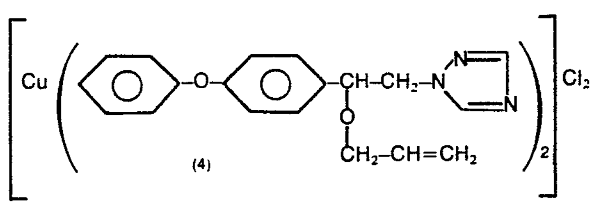 (4) | 26 |
| 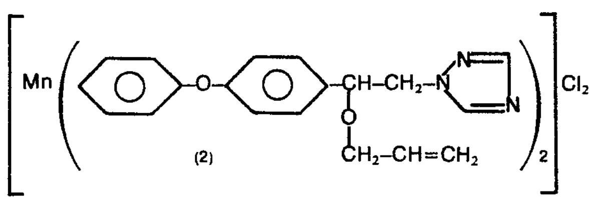 (2) | 34 |
| 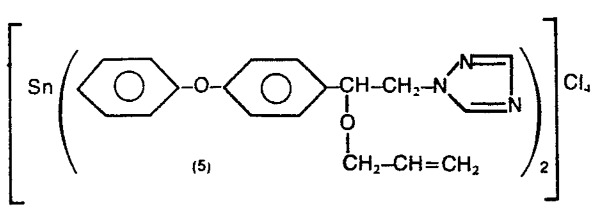 (5) | 30 |

Tabelle A (Fortsetzung)

Fusicladium-Test (Apfel)/Protektiv

| Wirkstoff | Befall in % bei einer Wirkstoffkonzentration von 0,0025% |
|---|---|
| 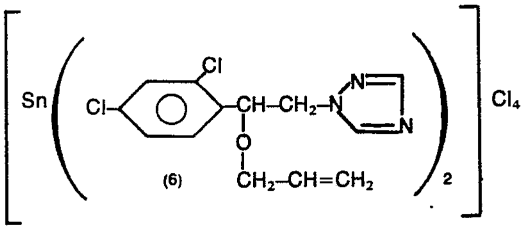 (6) | 34 |
| 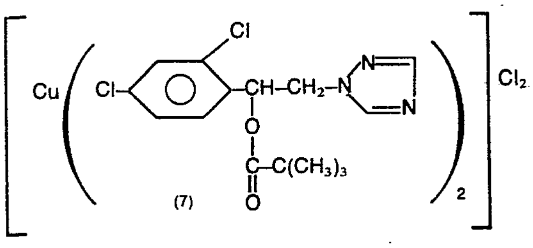 (7) | 11 |
| 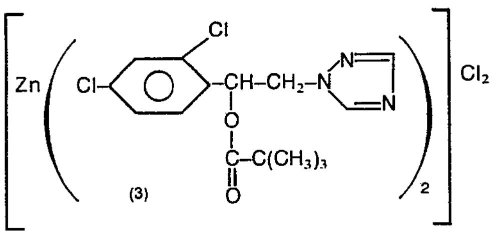 (3) | 21 |
| 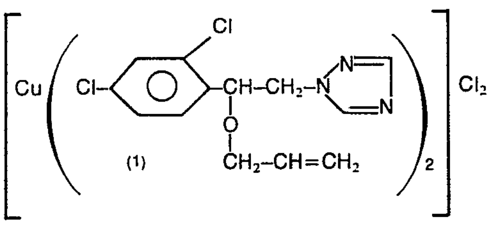 (1) | 15 |
| 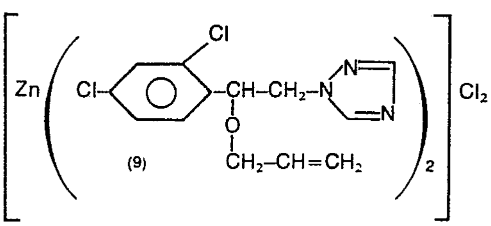 (9) | 16 |

Beispiel B

Podosphaera-Test (Apfel)/Protektiv

Lösungsmittel: 4,7 Gew.-Teile Aceton
Emulgator: 0,3 Gew.-Teile Alkylarylpolyglykolether
Wasser: 95,0 Gew.-Teile

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 Stunden bei 20°C und einer relativen Luftfeuchtigkeit von 70% im Gewächshaus. Anschliessend werden sie durch Bestäuben mit Konidien des Apfelmehltauerregers (Podosphaera leucotricha) inokuliert und in ein Gewächshaus mit einer Temperatur von 21 bis 23°C und einer relativen Luftfeuchtigkeit von ca. 70% gebracht.

10 Tage nach der Inokulation wird der Befall der Sämlinge bestimmt. Die erhaltenen Boniturwerte werden in % Befall umgerechnet. 0% bedeutet keinen Befall, 100% bedeutet, dass die Pflanzen vollständig befallen sind.

Wirkstoffe, Wirkstoffkonzentrationen und Ergebnisse gehen aus der nachfolgenden Tabelle hervor:

Tabelle B

Podosphaera-Test (Apfel)/Protektiv

| Wirkstoff | Befall in % bei einer Wirkstoffkonzentration von |
|---|---|
| | 0,0025% |
| 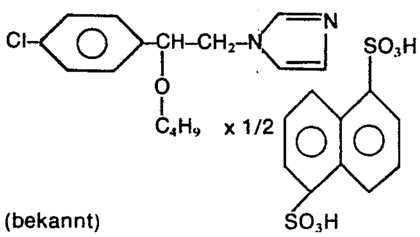 (bekannt) | 48 |
| 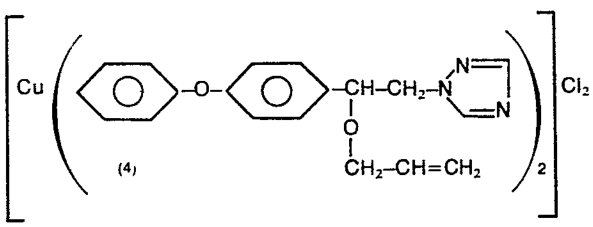 (4) | 22 |
| 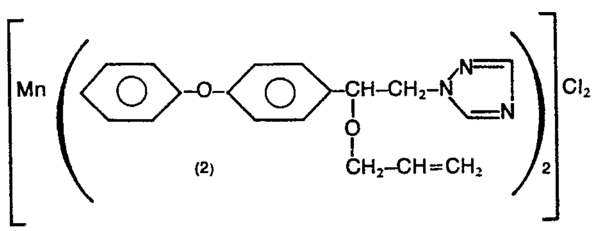 (2) | 29 |
| 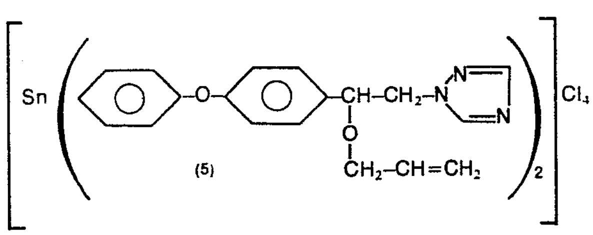 (5) | 7 |
| 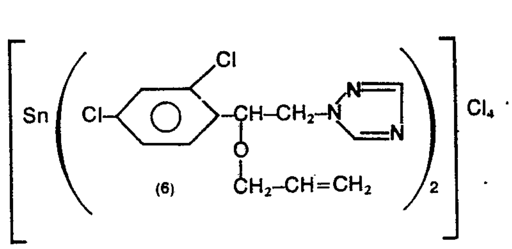 (6) | 0 |

Tabelle B (Fortsetzung)

Podosphaera-Test (Apfel)/Protektiv

| Wirkstoff | Befall in % bei einer Wirkstoffkonzentration von |
|---|---|
| 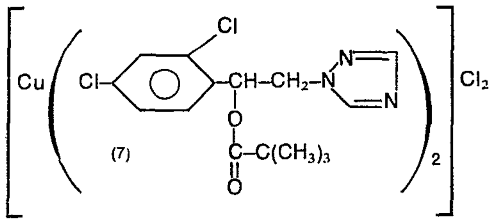 (7) | 0 |
| 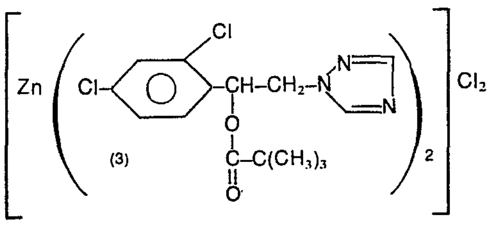 (3) | 0 |
| 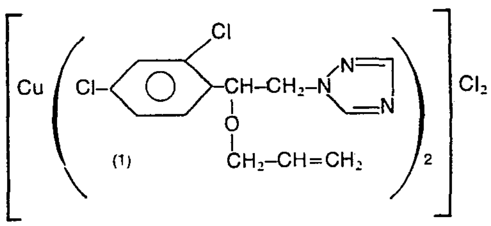 (1) | 1 |
| 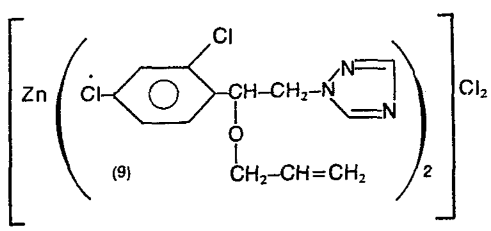 (9) | 0 |

Beispiel C

Sprossbehandlungs-Test / Getreidemehltau / protektiv (blattzerstörende Mykose)

Zur Herstellung einer zweckmässigen Wirkstoffzubereitung nimmt man 0,25 Gew.-Teile Wirkstoff in 25 Gew.-Teilen Dimethylformamid und 0,06 Gew.-Teilen Alkylaryl-polyglykolether auf und gibt 975 Gew.-Teile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte Amsel mit der Wirkstoffzubereitung taufeucht. Nach Antrocknen bestäubt man die Gerstenpflanzen mit Sporen von Erysiphe graminis var.hordei.

Nach 6 Tagen Verweilzeit der Pflanzen bei einer Temperatur von 21 bis 22°C und einer Luftfeuchtigkeit von 80 bis 90% wertet man den Besatz der Pflanzen mit Mehltaupusteln aus. Der Befallsgrad wird in Prozenten des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist umso wirksamer, je geringer der Mehltaubefall ist.

Wirkstoffe, Wirkstoffkonzentrationen in der Spritzbrühe und Befallsgrade gehen aus der nachfolgenden Tabelle hervor.

Tabelle C

Sprossbehandlungs-Test/Getreidemehltau/protektiv

| Wirkstoffe | Wirkstoffkonzentration in der Spritzbrühe in Gew.-% | Befall in % der unbehandelten Kontrolle |
|---|---|---|
| unbehandelt | – | 100 |
| 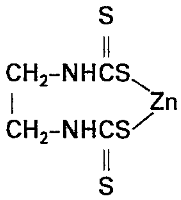 (bekannt) | 0,025 | 100 |
| 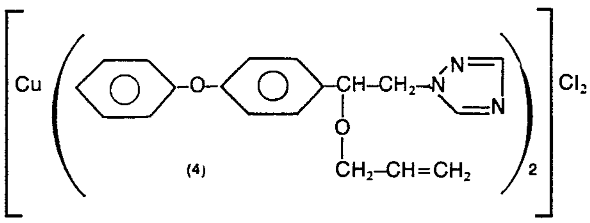 (4) | 0,025 | 12,5 |
| 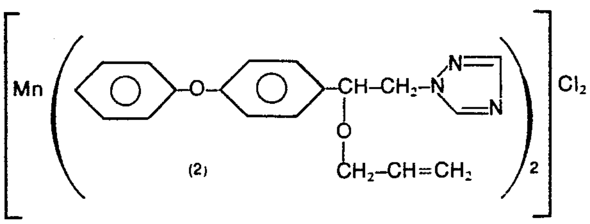 (2) | 0,025 | 8,8 |
| 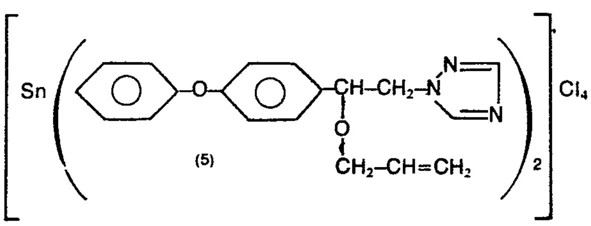 (5) | 0,025 | 3,8 |
| 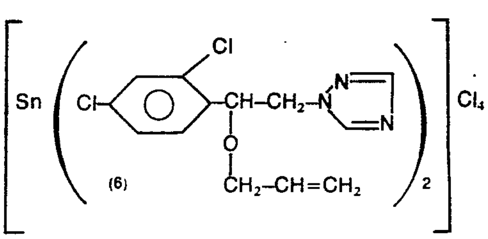 (6) | 0,025 | 0,0 |
| 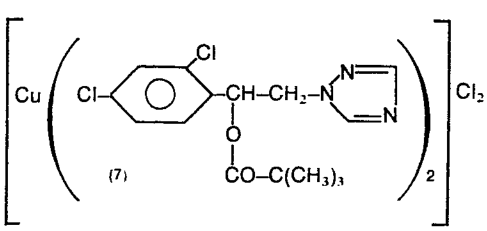 (7) | 0,025 | 0,0 |

Tabelle C (Fortsetzung)

| Wirkstoffe | Wirkstoffkonzentration in der Spritzbrühe in Gew.-% | Befall in % der unbehandelten Kontrolle |
|---|---|---|
| 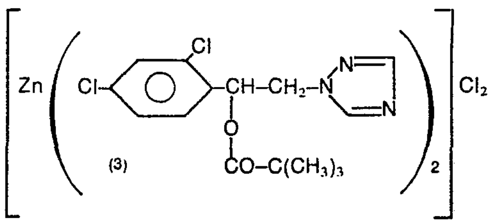 (3) | 0,025 | 0,0 |
| 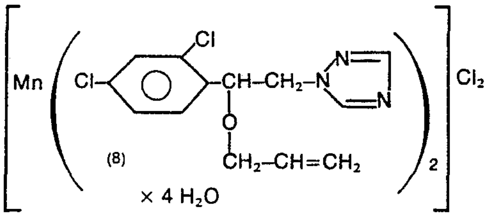 (8) | 0,025 | 0,0 |
| 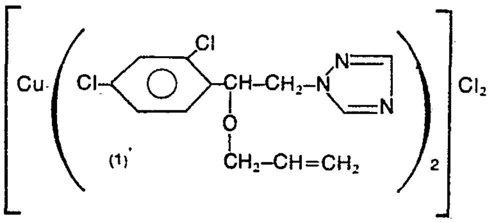 (1) | 0,025 | 0,0 |
| 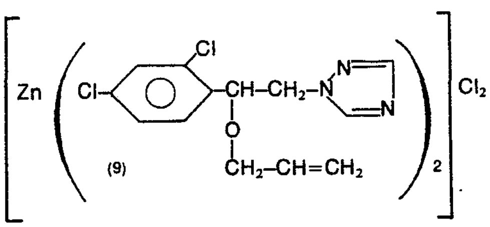 (9) | 0,025 | 0,0 |

Beispiel D

Gerstenmehltau-Test (Erysiphe graminis var. hordei)/ystemisch

(pilzliche Getreidesprosskrankheit)

Die Anwendung der Wirkstoffe erfolgt als pulverförmiges Saatgutbehandlungsmittel. Sie werden hergestellt durch Abstrecken des jeweiligen Wirkstoffes mit einem Gemisch aus gleichen Gew.-Teilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21 bis 22°C und 80 bis 90% rel. Luftfeuchte und 16stündiger Belichtung weiter kultiviert. Innerhalb von 6 Tagen bilden sich an den Blättern die typischen Mehltaupusteln aus.

Der Befallsgrad wird in Prozenten des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer je geringer der Mehltaubefall ist.

Wirkstoffe, Wirkstoffkonzentrationen im Saatgutbehandlungsmittel sowie dessen Aufwandmenge und der prozentuale Mehltaubefall gehen hervor aus der nachfolgenden Tabelle.

Tabelle D

Gerstenmehltau-Test (Erysiphe graminis var. hordei)/systemisch

| Wirkstoffe | Wirkstoff-konzentration im Beizmittel in Gew.-% | Beizmittel-aufwandmenge in g/kg Saatgut | Befall in % der unbe-handelten Kontrolle |
|---|---|---|---|
| ungebeizt | – | – | 100 |
| 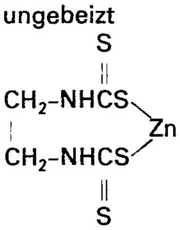 (bekannt) | 25 | 10 | 100 |
| 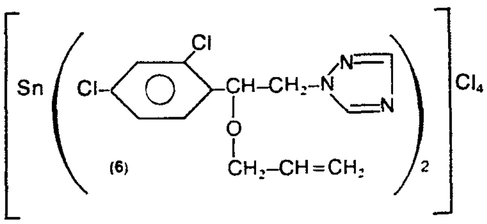 (6) | 25 | 10 | 0,0 |
| 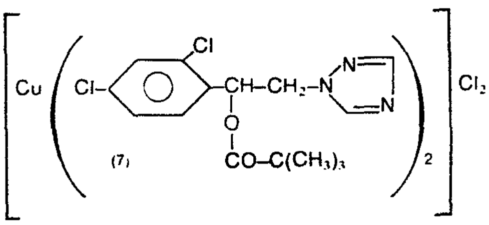 (7) | 25 | 10 | 0,0 |
| 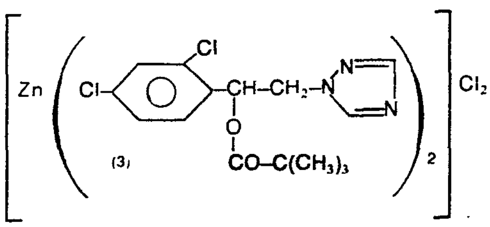 (3) | 25 | 10 | 0,0 |
| 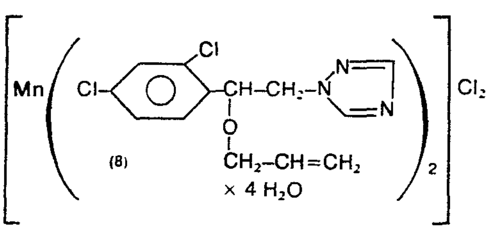 (8) | 25 | 10 | 0,0 |
| 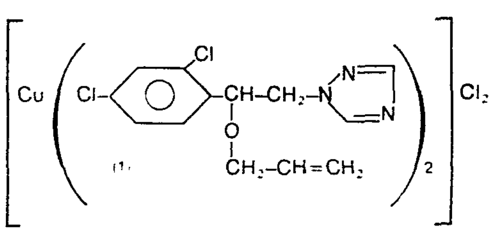 (1) | 25 | 10 | 0,0 |

Tabelle D (Fortsetzung)

Gerstenmehltau-Test (Erysiphe graminis var. hordei)/systemisch

| Wirkstoffe | Wirkstoffkonzentration im Beizmittel in Gew.-% | Beizmittelaufwandmenge in g/kg Saatgut | Befall in % der unbehandelten Kontrolle |
|---|---|---|---|
| 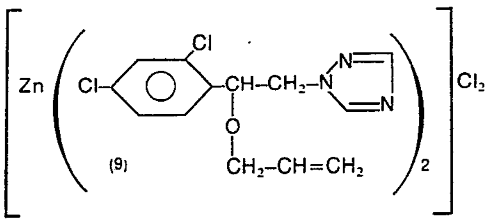 | 25 | 10 | 0,0 |

Beispiel E

Myzelwachstums-Test

Verwendeter Nährboden:

20 Gew.-Teile Agar-Agar
200 Gew.-Teile Kartoffeldekokt
5 Gew.-Teile Malz
15 Gew.-Teile Dextrose
5 Gew.-Teile Pepton
2 Gew.-Teile $Na_2HPO_4$
0,3 Gew.-Teile $Ca(NO_3)_2$

Verhältnis von Lösungsmittelgemisch zum Nährboden:

2 Gew.-Teile Lösungsmittelgemisch
100 Gew.-Teile Agarnährboden

Zusammensetzung Lösungsmittelgemisch:

0,19 Gew.-Teile DMF oder Aceton
0,01 Gew.-Teile Emulgator Alkylaryl-polyglykolether
1,80 Gew.-Teile Wasser

2 Gew.-Teile Lösungsmittelgemisch

Man vermischt die für die gewünschte Wirkstoffkonzentration im Nährboden nötige Wirkstoffmenge mit der angegebenen Menge des Lösungsmittelgemisches. Das Konzentrat wird im genannten Mengenverhältnis mit dem flüssigen, auf 42°C abgekühlten Nährboden gründlich vermischt und in Petrischalen mit einem Durchmesser von 9 cm gegossen. Ferner werden Kontrollplatten ohne Präparatbeimischung aufgestellt.

Ist der Nährboden erkaltet und fest, werden die Platten mit den in der Tabelle angegebenen Pilzarten beimpft und bei etwa 21°C inkubiert.

Die Auswertung erfolgt je nach der Wachstumsgeschwindigkeit der Pilze nach 4 bis 10 Tagen. Bei der Auswertung wird das radiale Myzelwachstum auf den behandelten Nährböden mit dem Wachstum auf den Kontrollnährböden verglichen. Die Bonitierung des Pilzwachstums geschieht mit folgenden Kennzahlen:

1 kein Pilzwachstum
bis 3 sehr starke Hemmung des Wachstums
bis 5 mittelstarke Hemmung des Wachstums
bis 7 schwache Hemmung des Wachstums
9 Wachstum gleich der unbehandelten Kontrolle

Wirkstoffe, Wirkstoffkonzentrationen und Resultate gehen aus der nachfolgenden Tabelle hervor:

Tabelle E

Mycelwachstums-Test/Pilze

| Wirkstoffe | Wirkstoffkonzentration ppm | Colletotrichum coffeanum | Botrytis cinerea | Verticillium alboatrum | Phytophthora cactorum | Pyricularia oryzae | Helminthosporum gramineum |
|---|---|---|---|---|---|---|---|
| 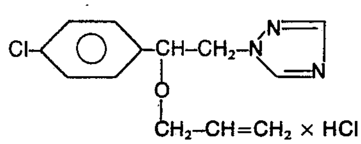 (bekannt) | 10 | 9 | 5 | 5 | 9 | 5 | 5 |

Tabelle E (Fortsetzung)
Mycelwachstums-Test/Pilze

| Wirkstoffe | Wirkstoff-konzen-tration ppm | Colleto-trichum coffeanum | Botrytis cinerea | Verticillium alboatrum | Phytophthora cactorum | Pyricularia oryzae | Helminthosporum gramineum |
|---|---|---|---|---|---|---|---|
| 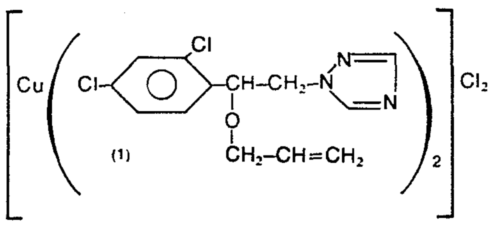 (1) | | 3 | - | 2 | 2 | 1 | 2 |
| 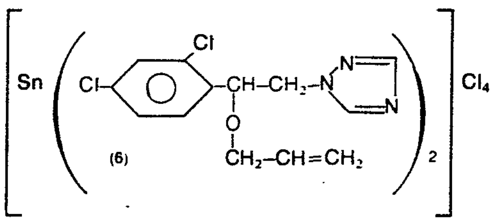 (6) | | - | 3 | 1 | - | 1 | 2 |
| 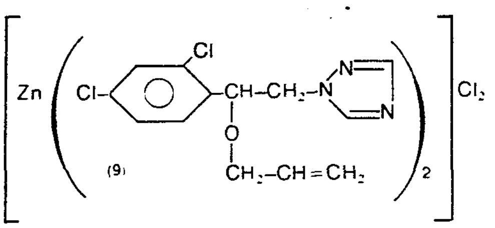 (9) | | 5 | - | 1 | 1 | 1 | 2 |

**Herstellungsbeispiele**

Beispiel 1

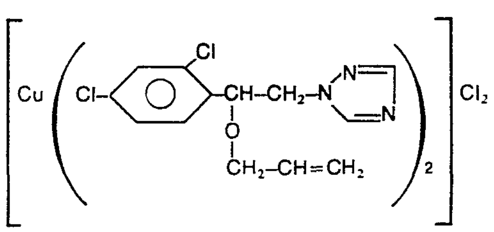

4,25 g (0,025 Mol) Kupferdichlorid ($CuCl_2 \times 2\,H_2O$) werden in 40 ml Wasser gelöst und unter Rühren zu 14,9 g (0,05 Mol) [1-(2,4-Dichlorphenyl)-2-(1,2,4-triazol-1-yl)-ethyl]-allyl-ether, gelöst in 100 ml Ethanol, getropft. Nach dreistündigem Rühren bei Raumtemperatur wird der Feststoff abgesaugt, mit Diethylether gewaschen und getrocknet. Man erhält 13,5 g (75% der Theorie) an Bis-[1-(2,4-Dichlorphenyl)-2-(1,2,4-triazol-1-yl)-ethyl]-allyl-ether)-kupfer (II)-chlorid vom Schmelzpunkt 158 bis 160°C.

Herstellung der Vorprodukte

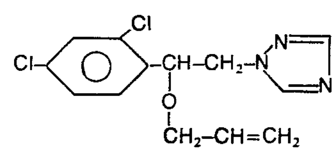

25,7 g (0,1 Mol) 1-Hydroxy-1-(2,4-dichlorphenyl)-2-(1,2,4-triazol-1-yl)-ethan werden in 125 ml Dioxan gelöst und unter Rühren zu einer Suspension von 4 g 80%igem Natriumhydrid in 150 ml Dioxan getropft. Danach wird eine Stunde unter Rückfluss erhitzt. Nach dem Abkühlen werden bei Raumtemperatur zu dem so erhaltenen Natriumsalz 22,1 g (0,1 Mol) Allylbromid zugegeben. Anschliessend erhitzt man 8 Stunden unter Rückfluss, lässt abkühlen und engt durch Abdestillieren des Lösungsmittels ein. Der Rückstand wird mit Wasser und Methylenchlorid versetzt, die organische Phase abgetrennt, über Natriumsulfat getrocknet und eingeengt. Es verbleiben 29,3 g [1-(2,4-Dichlorphenyl)-2-(1,2,4-triazol-1-yl)-ethyl]-allyl-ether mit dem Brechungsindex $n_D^{22}$ = 1,545, wobei die Ausbeute praktisch quantitativ ist.

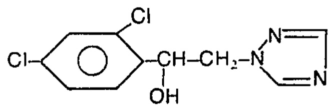

25,6 g (0,1 Mol) ω-(1,2,4-Triazol-1-yl)-2,4-dichloracetophenon werden in 300 ml Methanol gelöst und bei 5 bis 10°C unter Rühren portionsweise mit 4 g (0,1 Mol) Natriumborhydrid versetzt. Anschliessend wird eine Stunde bei Raumtemperatur nachgerührt und eine Stunde zum Sieden erhitzt. Nach Abdestillieren des Lösungsmittels wird der Rückstand mit 200 ml Wasser und 40 ml konzentrierter Salzsäure kurzzeitig erhitzt. Nachdem das Reaktionsgemisch mit Natronlauge alkalisch gemacht wurde, kann das feste Reaktionsprodukt abfiltriert werden. Nach dem Umkristallisieren aus Ligroin/Isopropanol erhält man 21,3 g (82% der Theorie) 1-Hydroxy-1-(2,4-dichlorphenyl)-2-(1,2,4-triazol-1-yl)-ethan vom Schmelzpunkt 90°C.

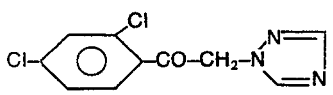

269 g (1 Mol) ω-Brom-2,4-dichloracetophenon werden in 250 ml Acetonitril gelöst. Diese Lösung tropft man zu einer unter Rückfluss siedenden Suspension von 69 g (1 Mol) 1,2,4-Triazol und 150 g Kaliumcarbonat in 2 l Acetonitril. Nach 20stündigem Erhitzen unter Rückfluss wird die erkaltete Suspension filtriert, das Filtrat vom Lösungsmittel befreit und der Rückstand mit Essigsäure aufgenommen, mit Wasser gewaschen, über Natriumsulfat getrocknet und vom Lösungsmittel befreit. Der Essigesterrückstand kristallisiert beim Versetzen mit Isopropanol aus.
Nach dem Umkristallisieren aus Ligroin/Isopropanol erhält man 154 g (60% der Theorie) ω-(1,2,4-Triazol-1-yl)-2,4-dichloracetophenon vom Schmelzpunkt 117°C.

Beispiel 2

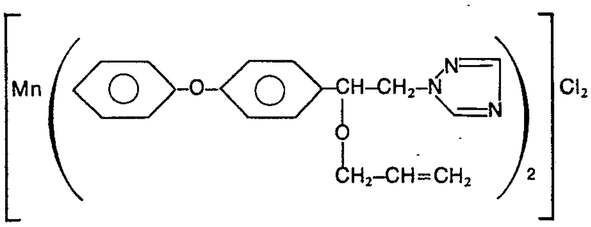

1,7 g (0,0083 Mol) Mangandichlorid werden in 40 ml Wasser gelöst und unter Rühren zu 5,3 g (0,0166 Mol) [1-(4-Phenoxyphenyl)-2-(1,2,4-triazol-1-yl)-ethyl]-allyl-ether, gelöst in 100 ml Ethanol, getropft. Man lässt 1,5 Stunden bei Raumtemperatur rühren. Das abgeschiedene Öl wird dekantiert und mit Petrolether verrieben, wobei es durchkristallisiert. Der Feststoff wird abgesaugt und getrocknet. Man erhält 5,5 g (87% der Theorie) an Bis-([1-(4-Phenoxyphenyl)-2-(1,2,4-triazol-1-yl)-ethyl]-allyl-ether)-mangan(II)-chlorid vom Schmelzpunkt 180 bis 200°C.

Beispiel 3

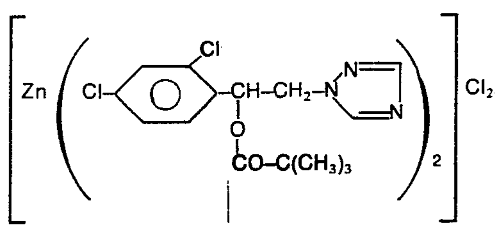

3,5 g (0,025 Mol) Zinkdichlorid werden in 40 ml Wasser gelöst und unter Rühren zu 17 g (0,05 Mol) 1-(2,4-Dichlorphenyl)-1-trimethylacetoxy-2-(1,2,4-triazol-1-yl)-ethan, gelöst in 100 ml Ethanol, getropft. Nach dreistündigem Rühren bei Raumtemperatur wird der Feststoff abgesaugt und getrocknet. Man erhält 13,1 g (72% der Theorie) Bis-[1-(2,4-Dichlorphenyl)-1-trimethylacetoxy-2-(1,2,4-triazol-1-yl)-ethan]-zink(II)-chlorid vom Schmelzpunkt 160°C.

Herstellung des Ausgangsproduktes

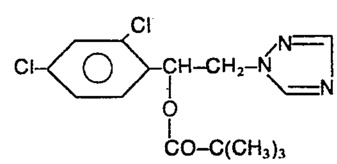

25,8 g (0,1 Mol) 1-(2,4-Dichlorphenyl)-1-hydroxy-2-(1,2,4-triazol-1-yl)-ethan werden in 200 ml Dioxan gelöst und unter Rühren zu einem Gemisch aus 6 g 80%igem Natriumhydrid und 100 ml Dioxan getropft. Danach wird ca. ½ Stunde unter Rückfluss erhitzt. Nach dem Abkühlen werden bei Raumtemperatur zu dem so erhaltenen Natriumsalz 24 g (0,2 Mol) Trimethylessigsäurechlorid in 100 ml Dioxan zugetropft. Man lässt über Nacht bei Raumtemperatur rühren, engt durch Abdestillieren des Lösungsmittels im Vakuum ein und nimmt den Rückstand in Chloroform auf. Die Chloroformlösung wird mit Wasser gewaschen, über Natriumsulfat getrocknet und wiederum eingeengt. Der Rückstand kann direkt weiter umgesetzt werden.
Analog den Beispielen 1 bis 3 werden die Verbindungen der folgenden Tabelle 4 erhalten.

Tabelle 4

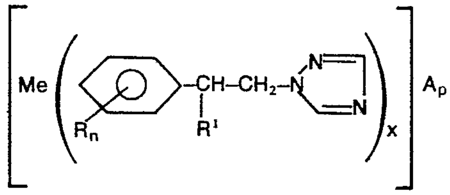

| Bsp. Nr. | Me | $R_n$ | $R^1$ | x | $A_p$ | Schmelzpunkt (°C) |
|---|---|---|---|---|---|---|
| 4 | Cu | 4-O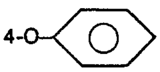 | $-O-CH_2-CH=CH_2$ | 2 | $Cl_2$ | 90-100 |
| 5 | Sn | 4-O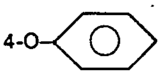 | $-O-CH_2-CH=CH_2$ | 2 | $Cl_4$ | 139-142 |
| 6 | Sn | 2,4-$Cl_2$ | $-O-CH_2-CH=CH_2$ | 2 | $Cl_4$ | 126-131 |
| 7 | Cu | 2,4-$Cl_2$ | $-O-CH_2-CH=CH_2$ | 2 | $Cl_2$ | 190-198 |
| 8 | Mn | 2,4-$Cl_2$ | $-O-CH_2-CH=CH_2$ | 2 | $Cl_2$ | 148-152 (×4 $H_2O$) |
| 9 | Zn | 2,4-$Cl_2$ | $-O-CH_2-CH=CH_2$ | 2 | $Cl_2$ | 128-130 |

In entsprechender Weise können die folgenden Verbindungen der allgemeinen Formel I hergestellt werden:

| Me | $R_n$ | $R^1$ | x | $A_n$ |
|---|---|---|---|---|
| Zn | 2,4-$Cl_2$ | 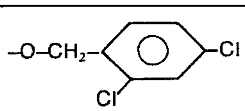 | 2 | $Cl_2$ |
| Zn | 2,4-$Cl_2$- | 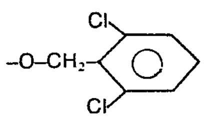 | 2 | $Cl_2$ |

**Patentansprüche**

1. Metallsalzkomplexe von 1-Phenyl-2-triazolyl-äthyl-Derivaten der allgemeinen Formel

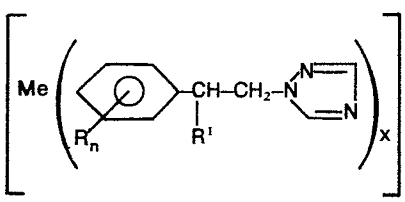 (I)

in welcher

R für Fluor, Chlor, Brom und für Alkyl mit 1 bis 4 Kohlenstoffatomen steht, ferner für gegebenenfalls durch Fluor, Chlor oder Brom substituiertes Phenyl oder Phenoxy steht,

$R^1$ für die Gruppierungen $-O-R^2$ oder $-O-CO-R^3$ steht, wobei $R^2$ und $R^3$ für Alkyl, Alkenyl und Alkinyl mit jeweils bis zu 4 Kohlenstoffatomen, ferner für gegebenenfalls im Arylteil durch Fluor, Chlor, Brom oder Alkyl mit 1 bis 4 Kohlenstoffatomen substituiertes Benzyl stehen,

A für ein Chlorid-, Nitrat- oder Sulfat-Anion steht,

Me für die Metalle Kupfer, Zink, Mangan, Magnesium, Zinn, Eisen und Nickel steht,

n für ganze Zahlen von 0 bis 3 steht,

p für ganze Zahlen von 1 bis 4 steht und

x für ganze Zahlen von 1 bis 4 steht.

2. Verfahren zur Herstellung von Metallsalzkomplexen von 1-Phenyl-2-triazolyl-äthyl-Derivaten, dadurch gekennzeichnet, dass man 1-Phenyl-2-triazolyl-äthyl-Derivate der Formel

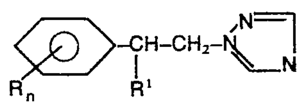 (II)

in welcher

R, $R^1$ und n die in Anspruch 1 angegebene Bedeutung haben,

mit Metallsalzen der Formel

$MeA_p \times k\,H_2O$ (III)

in welcher

k für beliebige Zahlen von 0 bis 12 steht und

Me, A und p die an Anspruch 1 angegebene Bedeutung haben,

in Gegenwart eines Lösungsmittels umsetzt.

3. Fungizide Mittel, gekennzeichnet durch einen Gehalt an mindestens einem Metallkomplex von 1-Phenyl-2-triazolyl-äthyl-Derivat gemäss Anspruch 1.

4. Verwendung von Metallsalzkomplexen von 1-Phenyl-2-triazolyl-äthyl-Derivaten gemäss Anspruch 1 zur Bekämpfung von Pilzen.

**Revendications**

1. Complexes de sels métalliques de dérivés de 1-phényl-2-triazolyl-éthyle de formule générale:

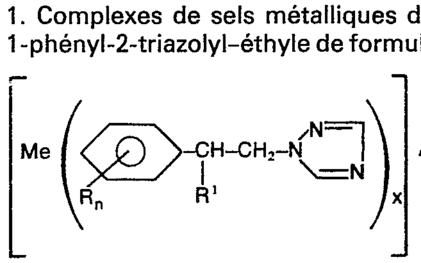 (I)

dans laquelle

R représente du fluor, chlore, brome et un alcoyle ayant 1 à 4 atomes de carbone, en outre un phényl ou phénoxy éventuellement substitué par du fluor, chlore ou brome,

$R^1$ représente les groupements $-O-R^2$ ou $-O-CO-R^3$, où $R^2$ et $R^3$ représentent un alcoyle, alcényle et alcinyle ayant chaque fois jusqu'à 4 atomes de carbone, en outre un benzyle éventuellement substitué dans la partie aryle par du fluor, chlore ou brome ou un alcoyle ayant 1 à 4 atomes de carbone,

A représente un anion chlorure, nitrate ou sulfate,

Me représente les métaux cuivre, zinc, manganèse, magnésium, étain, fer et nickel,

n représente les nombres entiers de 0 à 3,

p représente les nombres entiers de 1 à 4 et

x représente les nombres entiers de 1 à 4.

2. Procédé de préparation de complexes de sels métalliques de dérivés de 1-phényl-2-triazolyl-éthyle, caractérisé en ce qu'on fait réagir des dérivés de 1-phényl-2-triazolyl-éthyle de formule:

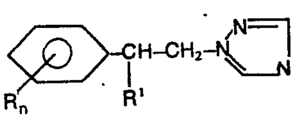

(II)

dans laquelle

R, $R^1$ et n ont la signification indiquée à la revendication 1,

avec des sels métalliques de formule:

$$MeA_p \times k\,H_2O \qquad (III)$$

dans laquelle

k représente des nombres quelconques de 0 à 12 et

Me, A et p ont la signification indiquée à la revendication 1,

en présence d'un solvant.

3. Agents fongicides, caractérisés par une teneur en au moins un complexe métallique d'un dérivé de 1-phényl-2-triazolyl-éthyle selon la revendication 1.

4. Utilisation de complexes de sels métalliques de dérivés de 1-phényl-2-triazolyl-éthyle selon la revendication 1 pour combattre les champignons.

**Claims**

1. Metal salt complexes of 1-phenyl-2-triazolyl-ethyl derivates of the general formula

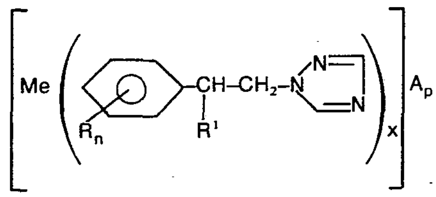

(I)

in which

R represents fluorine, chlorine, bromine and alkyl with 1 to 4 carbon atoms, also phenyl or phenoxy optionally substitute by fluorine, chlorine or bromine,

$R^1$ represents the groupings $-O-R^2$ or $-O-CO-R^3$, wherein $R^2$ and $R^3$ represent alkyl, alkenyl and alkinyl with in each case up to 4 carbon atoms, also benzyl optionally substituted in the aryl part by fluorine, chlorine, bromine or alkyl with 1 to 4 carbon atoms,

A represents a chloride, nitrate or sulphate anion,

Me represents the metalls copper, zinc, manganese, magnesium, tin, iron and nickel,

n represents integers from 0 to 3,

p represesnets integers from 1 to 4 and

x represents integers from 1 to 4.

2. Process for the preparation of metal salt complexes of 1-phenyl-2-triazolyl-ethyl derivatives, characterised in that 1-phenyl-2-triazolyl-ethyl derivatives of the formula

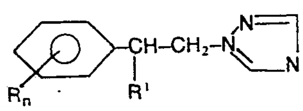

(II)

in which

R, $R^1$ and n have the meaning given in claim 1,

are reacted with metal salts of the formula

$$MeA_p \times k\,H_2O \qquad (III)$$

in which

k represents any numbers from 0 to 12 and

Me, A and p have the meaning given in claim 1,

in the presence of a solvent.

3. Fungicidal compositions, characterised in that they contain at least one metal complex of the 1-phenyl-2-triazolyl-ethyl derivatives according to claim 1.

4. Use of metal salt complexes of 1-phenyl-2-triazolylethyl derivatives according to claim 1 for combating fungi.